# EUROPEAN PATENT APPLICATION

(11) **EP 4 445 723 A1**
(43) Date of publication of application: **16.10.2024**
(21) Application number: 24169380.3
(22) Date of filing: 10.04.2024
(51) Int. Cl.: A01H 1/04, A01H 1/00, A01H 5/12, A01H 6/02, C12Q 1/6895

(54) **METHODS AND COMPOSITIONS FOR PERONOSPORA RESISTANCE IN SPINACH**

(30) Priority: 14.04.2023 US 202363496102 P
(71) Applicant: Seminis Vegetable Seeds, Inc., St. Louis MO 63167 (US)
(72) Inventor: GOOSSENS, Rudolphus Josephus Maria, St. Louis, Missouri, 63167 (US); KNISKERN, Joel M., St. Louis, Missouri, 63167 (US); MEEUWSEN, Johannes Theodorus Hendrikus, St. Louis, Missouri, 63167 (US); OORSCHOT, Inge van, St. Louis, Missouri, 63167 (US); SELBY, Jessica Packard, St. Louis, Missouri, 63167 (US)
(74) Representative: BIP Patents

(57) **Abstract**

Spinach (*Spinacia oleracea)* plants exhibiting resistance to downy mildew disease caused by *Peronospora effusa* are provided, together with methods of producing, identifying, or selecting plants or germplasm with a downy mildew resistance phenotype. Such plants include spinach plants comprising introgressed genomic regions conferring pest resistance. Compositions, including novel polymorphic markers for detecting plants comprising introgressed loci, are further provided.

## Description

### CROSS-REFERENCE TO RELATED APPLICATIONS

This application claims priority to U.S. Provisional Patent Application No. 63/496,102, filed April 14, 2023, the content of which is incorporated herein by reference in its entirety.

### INCORPORATION OF SEQUENCE LISTING

The sequence listing that is contained in the file named "BCS226256 US01.xml," which is 12 kilobytes as measured in Microsoft Windows operating system and was created on March 21, 2024, is filed electronically herewith and incorporated herein by reference.

### FIELD OF THE INVENTION

The present disclosure relates to the field of plant breeding and, more specifically, to methods and compositions for producing spinach plants with desired resistance to downy mildew.

### BACKGROUND OF THE INVENTION

Plant disease resistance is an important trait in plant breeding, particularly for production of food crops. Downy mildew, caused by the plant fungal pathogen *Peronospora effusa,* is an economically important disease of spinach worldwide, particularly for *Spinacia oleracea,* the most commonly cultivated spinach variety. As of December 2022, a total of 19 races of *Peronospora effusa* are officially recognized, although new isolates are currently being discovered and named each year by the International Working Group Peronospora (IWGP). To date, it has been believed that resistance to downy mildew in spinach was race-specific. The ability of new strains of the pathogen to overcome resistance in spinach plants thus makes the development of spinach varieties with effective levels of resistance to *Peronospora effusa* challenging and increasingly important.

### SUMMARY OF THE INVENTION

In one aspect, the present disclosure provides a *Spinacia oleracea* plant comprising a recombinant chromosomal segment from *Spinacia tetrandra* on chromosome 3, wherein said recombinant chromosomal segment comprises an allele that confers broad-spectrum resistance to *Peronospora effusa* relative to a plant lacking said recombinant chromosomal segment. In some embodiments, said recombinant chromosomal segment comprises a marker locus selected from the group consisting of marker M1 (SEQ ID NO:1), marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), marker M8 (SEQ ID NO:8), and marker M9 (SEQ ID NO:9). In some embodiments, said recombinant chromosomal segment comprises a marker locus selected from the group consisting of marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7) and marker M8 (SEQ ID NO:8). In other embodiments, the broad-spectrum resistance comprises resistance to at least races Pe: 14, Pe: 15, Pe: 16, and Pe: 17 of *Peronospora effusa.* In further embodiments, the broad-spectrum resistance further comprises resistance to Pe:19 of *Peronospora effusa.* In further embodiments, the broad-spectrum resistance further comprises resistance to Pe:13 of *Peronospora effusa.* In some embodiments, the broad-spectrum resistance further comprises resistance to at least one *Peronospora effusa* race selected from the group consisting of: Pe:6 and Pe:8. In yet further embodiments, the broad-spectrum resistance further comprises resistance to at least one *Peronospora effusa* isolate selected from the group consisting of: 254 and 381. In further embodiments, the broad-spectrum resistance further comprises resistance to at least one *Peronospora effusa* race selected from the group consisting of: Pe:6, Pe:8, Pe:13, Pe:18, and Pe:19. In some embodiments, the plant is defined as an inbred plant or a hybrid plant. In other embodiments, the plant is defined as an agronomically elite plant. In further embodiments, a representative sample of seed comprising said recombinant chromosomal segment has been deposited under NCMA Accession No. 202210003. Seed that produce the *Spinacia oleracea* plants of the present invention are also provided herein.

In addition, the present disclosure provides a plant part of a *Spinacia oleracea* plant comprising a recombinant chromosomal segment from *Spinacia tetrandra* on chromosome 3, wherein said recombinant chromosomal segment comprises an allele that confers broad-spectrum resistance to *Peronospora effusa* relative to a plant lacking said recombinant chromosomal segment. In some embodiments, the plant part is an embryo, a meristem, a cotyledon, pollen, a leaf, an anther, a root, a pistil, a flower, and a cell. In other embodiments, a tissue culture comprising a cell from a plant part of a *Spinacia oleracea* plant comprising a recombinant chromosomal segment from *Spinacia tetrandra* on chromosome 3, wherein said recombinant chromosomal segment comprises an allele that confers broad-spectrum resistance to *Peronospora effusa* relative to a plant lacking said recombinant chromosomal segment, wherein the cell comprises the recombinant chromosomal segment.

Any plant disclosed herein may as an alternative to breeding techniques involving crossing and selection also be obtained exclusively by non-essentially biological processes. In other words, in one embodiment relating to *Spinacia oleracea* plants according to the invention, these exclude plants obtained exclusively by essentially biological processes. Such processes include introduction of traits via site-specific modification, altering or introducing a single genetic locus or introducing a transgene into the genome of a variety or progenitor thereof, all as described further below.

In another aspect, the present disclosure provides a recombinant DNA segment (which may be referred to as a nucleic acid molecule) comprising an allele from *Spinacia tetrandra* conferring broad-spectrum resistance to *Peronospora effusa* and lacking all or some genetic loci that are genetically linked thereto in *Spinacia tetrandra.* In some embodiments, the recombinant DNA segment comprises the sequence of marker M1 (SEQ ID NO:1), marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), marker M8 (SEQ ID NO:8), or marker M9 (SEQ ID NO:9). In other embodiments, the recombinant DNA segment comprises the sequence of marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), or marker M8 (SEQ ID NO:8). In other embodiments, the recombinant DNA segment is further defined as comprising at least a first genetic locus from *Spinacia oleracea.* In certain embodiments, a representative sample of seed comprising said DNA segment from *Spinacia tetrandra* conferring broad-spectrum resistance to *Peronospora effusa* has been deposited under NCMA Accession No. 202210003. In further embodiments, the recombinant DNA segment is further defined as comprised within a plant, plant part, plant cell, or seed.

In yet another aspect, the present disclosure provides a nucleic acid molecule comprising an allele conferring resistance to *Peronospora effusa,* which nucleic acid molecule is obtainable or can be obtained from *S*. *oleracea seed* deposited under NCMA Accession No. 202210003 and which nucleic acid molecule comprises at least one marker selected from the group consisting of M1, M2, M3, M4; M5, M6, M7, M8, and M9. In one embodiment, the at least one marker is selected from the group consisting of M2, M3, M4; M5, M6, M7 and M8. In yet another embodiment, the allele conferring resistance to *Peronospora effusa* is obtained from a *S. tetrandra* species. In yet another embodiment, the nucleic acid molecule at its 5' or 3' end further comprises nucleic acid sequences originating from *S. oleracea.*

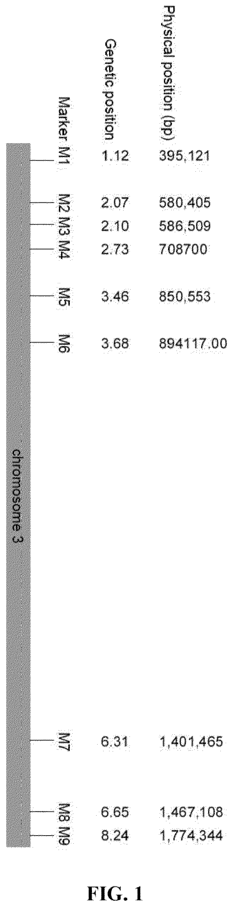

In yet another aspect, the present disclosure provides a nucleic acid marker having a nucleic acid sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, and SEQ ID NO:9. In one embodiment the nucleic acid sequence has one or more of the following sequences: SEQ ID NO 1, comprising a G at position 151; SEQ ID NO 2, comprising a G at position 151; SEQ ID NO 3, comprising a A at position 151; SEQ ID NO 4, comprising a G at position 151; SEQ ID NO 5, comprising a T at position 151; SEQ ID NO 6, comprising a T at position 151; SEQ ID NO 7, comprising a A at position 151; SEQ ID NO 8, comprising a T at position 151; and SEQ ID NO 9, comprising a T at position 151. The present invention also relates to a combination of nucleic acid markers as described above, wherein the combination comprises at least two markers or at least three markers selected from the group consisting of M2, M3, M4; M5, M6, M7 and M8. In this respect, a "combination of nucleic acid markers" relates to at least two markers as described that are present in one single nucleic acid molecule or on one single chromosome 3 or in one single S. oleracea genome, preferably in the assembly as described herein, i.e. in the sequence M1 through M9, see Figure 1. Such combination of nucleic acid markers enable for the identification and/or selection of alleles or nucleic acids that, when comprised in an *S. oleracea* plant, confer resistance to *Peronospora effusa* as described herein.

In yet another aspect, the present disclosure provides a method for producing an elite *Spinacia oleracea* plant with broad-spectrum resistance to *Peronospora effusa* comprising introgressing into said plant a *Peronospora effusa* resistance allele within a recombinant chromosomal segment flanked in the genome of said plant by marker M1 (SEQ ID NO:1) and marker M9 (SEQ ID NO:9) on chromosome 3 or flanked in the genome of said plant by marker M2 (SEQ ID NO: 2) and marker M8 (SEQ ID NO: 8) on chromosome 3, wherein said resistance allele confers to said plant broad-spectrum resistance to *Peronospora effusa* relative to a plant lacking said allele, and wherein said introgressing comprises marker-assisted selection. In some embodiments, said introgressing comprises: a) crossing a plant comprising said recombinant chromosomal segment with itself (also known as selfing) or with a second *Spinacia oleracea* plant of a different genotype to produce one or more progeny plants; and b) selecting a progeny plant comprising said recombinant chromosomal segment. In other embodiments, selecting a progeny plant comprises detecting nucleic acids comprising marker M1 (SEQ ID NO: 1), marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), marker M8 (SEQ ID NO:8), or marker M9 (SEQ ID NO:9). In other embodiments, selecting a progeny plant comprises detecting nucleic acids comprising marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), or marker M8 (SEQ ID NO:8), or combinations thereof. In further embodiments, said introgressing further comprises backcrossing or assaying for said broad-spectrum resistance to *Peronospora effusa.* In yet further embodiments, the progeny plant is an F₂-F₆ progeny plant. The present disclosure further provides *Spinacia oleracea* plants obtainable by the methods provided herein.

In another aspect, the present disclosure provides a method of selecting a *Spinacia oleracea* plant exhibiting broad-spectrum resistance to *Peronospora effusa,* comprising: a) crossing a *Spinacia oleracea* plant comprising a recombinant chromosomal segment from *Spinacia tetrandra* on chromosome 3, wherein said recombinant chromosomal segment comprises an allele that confers broad-spectrum resistance to *Peronospora effusa* relative to a plant lacking said recombinant chromosomal segment with itself (also referred to as selfing) or with a second *Spinacia oleracea* plant of a different genotype to produce one or more progeny plants; and b) selecting a progeny plant comprising said recombinant chromosomal segment. In some embodiments, selecting the progeny plant comprises detecting a marker locus genetically linked to said recombinant chromosomal segment. In other embodiments, selecting the progeny plant comprises detecting a marker locus within or genetically linked to a chromosomal segment flanked in the genome of said plant by marker M1 (SEQ ID NO:1) and marker M9 (SEQ ID NO:9) on chromosome 3. In some embodiments, selecting a progeny comprises detecting nucleic acids comprising marker M1 (SEQ ID NO:1), marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), marker M8 (SEQ ID NO:8), or marker M9 (SEQ ID NO:9). In some other embodiments, selecting a progeny comprises detecting nucleic acids comprising at least one of the following markers: M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), or marker M8 (SEQ ID NO:8). In certain embodiments, said progeny plant is an F₂-F₆ progeny plant. In further embodiments, producing said progeny plant comprises backcrossing.

In another aspect, the present disclosure provides a method of selecting a *Spinacia oleracea* plant or *Spinacia oleracea* seed, comprising a) detecting in a population of *Spinacia oleracea* plants or *Spinacia oleracea* seeds a *Spinacia oleracea* plant or *Spinacia oleracea* seed comprising an allele on chromosome 3 that confers broad-spectrum resistance to *Peronospora effusa* relative to a *Spinacia oleracea* plant lacking said allele and b) selecting said *Spinacia oleracea* plant or *Spinacia oleracea* seed comprising said allele on chromosome 3 that confers broad-spectrum resistance to *Peronospora effusa.* In some embodiments, the allele is associated with at least one of the following markers: M1, M2, M3, M4, M5, M6, M7, M8 and M9. In yet another embodiment, the allele is associated with at least one of the following markers: M2, M3, M4, M5, M6, M7 and M8. In a further embodiment, M1 comprises a G at position 151 in SEQ ID NO 1; M2 comprises a G at position 151 in SEQ ID NO 2; M3 comprises an A at position 151 in SEQ ID NO 3; M4 comprises a G at position 151 in SEQ ID NO 4; M5 comprises a T at position 151 in SEQ ID NO 5; M6 comprises a T at position 151 in SEQ ID NO 6; M7 comprises a A at position 151 in SEQ ID NO 7; M8 comprises a T at position 151 in SEQ ID NO 8; M9 comprises a T at position 151 in SEQ ID NO 9. In other embodiments of this aspect, the broad-spectrum resistance that is conferred comprises resistance to at least races Pe:14, Pe:15, Pe:16, and Pe:17 of *Peronospora effusa.* In further embodiments, the broad-spectrum resistance that is conferred further comprises resistance to Pe:19 of *Peronospora effusa.* In further embodiments, the broad-spectrum resistance that is conferred further comprises resistance to Pe:13 of *Peronospora effusa.* In some embodiments, the broad-spectrum resistance that is conferred further comprises resistance to at least one *Peronospora effusa* race selected from the group consisting of: Pe:6 and Pe:8. In yet further embodiments, the broad-spectrum resistance further comprises resistance to at least one *Peronospora effusa* isolate selected from the group consisting of: 254 and 381. In further embodiments, the broad-spectrum resistance comprises resistance to at least one *Peronospora effusa* race selected from the group consisting of: Pe:6, Pe:8, Pe:13, Pe:18, and Pe:19. In a further embodiment, the *Spinacia oleracea* plant or *Spinacia oleracea* seed is a cultivated plant or a seed of a cultivated plant. In yet a further embodiment, the *Spinacia oleracea* plant or *Spinacia oleracea* seed is an elite plant or a seed of an elite plant. In yet another aspect, the present disclosure provides a plant identifiable by the above-referenced method of screening.

In yet a further aspect, the present disclosure provides a food product comprising the harvested leaves of a *Spinacia oleracea* plant comprising a recombinant chromosomal segment from *Spinacia tetrandra* on chromosome 3, wherein said recombinant chromosomal segment comprises an allele that confers broad-spectrum resistance to *Peronospora effusa* relative to a plant lacking said recombinant chromosomal segment.

It is to be understood that all preferred embodiments disclosed in connection with one aspect of the present invention may be applied to any other aspect. This is in particular the case if the same terminology is applied. Accordingly, as an example, disclosure describing markers M1 to M9 applied to one aspect may be applied to any other aspect if markers are referenced therein. Furthermore, disclosure of the DETAILED DESCRIPTION OF THE INVENTION may explicitly be combined with any of the above embodiments if applicable.

### BRIEF DESCRIPTION OF THE DRAWINGS

**FIG. 1****:** Shows an overview of marker positions and chromosomal region on chromosome 3 that confers resistance to *Peronospora effusa* in spinach.

### DETAILED DESCRIPTION OF THE INVENTION

As plant growers face devastating plant disease outbreaks, sources of new and diverse genetic variation is essential for crop improvement in plant breeding programs. This is the case with downy mildew disease caused by the obligate oomycete *Peronospora effusa* (formerly known as *P. farinosa* f. sp. *spinaciae*), which remains the most economically important disease in spinach. In cultivated spinach production, downy mildew is managed using cultivars with genetic resistance and/or regular fungicide applications. However, the increasing demand from consumers for organic spinach, which cannot be treated with synthetic chemical fungicides, has resulted in an increase in demand by growers for cultivars that have genetic resistance to all officially recognized *Peronospora effusa* races. However, it is becoming increasingly difficult for breeders to keep up with development of such varieties due to the high frequency at which new pathogenic races overcome the existing genetic resistance used in commercial cultivars and to the limited availability of alleles that provide durable resistance. One of the major goals of crop improvement is to develop varieties with broad-spectrum resistance to pathogens. Broad-spectrum resistance refers to resistance against two or more types of pathogen species or the majority of races of the same pathogen species (Kou and Wang, 2010). Most of the major resistance genes identified to date only confer race-specific resistance to their adapted pathogens. Although these resistance genes are effective for a specific pathogen, their durability in the field is typically short due to the high frequency at which the pathogen population breaks through the resistance.

Downy mildew resistant spinach cultivars are bred using single dominant RPF (Resistance to *Peronospora farinosa*) genes, each gene being resistant to a particular race of *Peronospora effusa.* These resistance genes have been mapped to the proximal end of chromosome 3 and localized to the interval spanning from roughly 500kb to 1.5Mb. RPF gene stacking is commonly used in spinach cultivars to provide resistance to a specific subset of races. Newly identified *Peronospora effusa* isolates are assigned to different races based on their ability to infect a standardized set of differential spinach lines developed by the International Seed Federation (ISF). These spinach lines contain one or more resistance loci that are known to effectively contain the known *Peronospora effusa* races (Feng *et al.*, 2018). If a newly identified *Peronospora effusa* isolate is able to overcome host resistance as represented by the differential spinach lines, it is considered to be a new race. The new isolates are evaluated on an annual basis by the International Working Group on *Peronospora* (IWGP) and if recognized assigned a race number. The extensive and increasing use of commercial spinach varieties with genetic-based resistance to *Peronospora effusa* has likely led to a tremendous increase in the number of identified races. Prior to 1990, only three races of the *Peronospora effusa* were known (Koike *et al.*, 1992). However, as of 2022, the International Working Group on *Peronospora* (IWGP) had recognized 19 unique and economically important races of *Peronospora effusa.* As of 2023, there are two isolates with novel virulence patterns, currently designated as "254" and "381," which may soon be designated as new races.

Spinach is a wind-pollinated dioecious species which results in high diversity across the species. As such, spinach accessions are genetically heterogeneous, showing segregation and variation between and within accessions. Domestication of a wild species results in future plants having a fixed subset of variation from the original wild plants and overall limited genetic heterogeneity. Though it is challenging, it is therefore important to find and identify genetic variation within and among wild accessions. Wild accessions are typically obtained on expedition trips to various geographic regions. For expedition trips, material is generally collected by gathering seeds of several plants which are located in proximity at a location. As these are wild species, the plants are not the same and their genetic background is not uniform. In addition, since the plants are naturally pollinated by wind or insects, the pollen that fertilizes the flowers can have come from anywhere in the surrounding environment or even further afield. Based on these factors, the genetic composition of a single seed bag containing a wild *Spinacia* sp. accession is generally a bulk seed collection which has genetic variations within the individual seeds of the accession.

Wild spinach accessions have especially been valuable sources of novel resistance alleles against diseases caused by bacteria, fungi, and viruses. The two main wild species, *S*. *turkestanica* and *S*. *tetrandra*, are assigned to the primary gene pool of cultivated spinach, although reduced fertility has been observed in the hybrid offspring of *S. oleracea* and *S. tetrandra.* Furthermore, identification and characterization of traits by possessed these species has been difficult due to the limited molecular and genomic resources and limited number of wild accessions available in public germplasm repositories. As such, studies to identify and map valuable traits in spinach have been limited when compared to progress made in other vegetable crops.

In accordance with the present disclosure, one of skill in the art may identify a candidate germplasm source possessing a desirable downy mildew -resistant phenotype as described herein, such as from a given *S*. *tetrandra* accession. As *S. tetrandra* is a wild species, accessions can be collected from regions in which it is normally found, such as in Central Asia, including Uzbekistan and Tajikistan. In addition, accessions of *S. tetrandra* are available from genebanks including Centre for Genetic Resources, the Netherlands (CGN), Wageningen, the Netherlands and the National Plant Germplasm System of the U.S. Department of Agriculture (USDA). A collection trip for *S. tetrandra* was undertaken in 2011 by CGN and resulted in an increase in the total global accessions of *S. tetrandra* to 59 (*see,* for example, Kik *et al.*, 2011, The CGN Spinach Collection: Overview and Recent Collecting Expeditions, available at spinach.uark.edu/Session%20II%20PDFs/Chirs%20Kik.pdf). One embodiment of the invention comprises using the materials and methods of the present disclosure to obtain a locus conferring broad-spectrum resistance to downy mildew from any additional accessions of *S. tetrandra.* Using the information set forth herein, including, but not limited to the polymorphic markers provided herein, the downy mildew resistance from *S. tetrandra* can be introgressed into *S. oleracea* varieties without the poor agronomic properties otherwise associated with *S. tetrandra.*

The present disclosure represents a significant advance in that it provides, in one embodiment, downy mildew resistance in spinach plants conferred by a novel recombinant chromosomal segment from *S. tetrandra* comprising the QTL, as well as methods for the production thereof. Novel markers for the new locus are provided herein, allowing the locus to be accurately introgressed and tracked during development of new varieties. As such, the present disclosure permits introgression, or any other kind of transfer including new breeding technologies involving genetic engineering, of the downy mildew resistance locus derived from *S. tetrandra* into potentially any desired elite spinach variety. Although accession GB1860 was previously identified as a source of a downy mildew resistance allele (WO 2015054339 A1), the present inventors have found, through haplotype analysis, that the accession was a bulk population that included at least six distinct haplotypes and the resistance allele described herein is distinct - structurally and functionally - from downy mildew resistance alleles that have previously been described. The present disclosure provides a novel downy mildew resistance allele from *S. tetrandra* accession GB1860 that is distinct from the alleles described previously and has a unique resistance pattern.

In some embodiments, the introgressed downy mildew resistance allele is defined as located within a recombinant chromosomal segment from *S. tetrandra* flanked by marker M1 (SEQ ID NO:1) and marker M9 (SEQ ID NO:9) on chromosome 3. In other embodiments, such a segment can comprise one or more of marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), and marker M8 (SEQ ID NO:8). Marker M1 comprises a SNP change from A to G at 395,121 bp of the public *S. oleracea* reference genome v1, marker M2 comprises a SNP change from T to G at 580,405 bp of the public *S. oleracea* reference genome v1, marker M3 comprises a SNP change from G to A at 586,509 bp of the public *S. oleracea* reference genome v1, marker M4 comprises a SNP change from T to G at 708,700 bp of the public *S*. *oleracea* reference genome v1, marker M5 comprises a SNP change from G to T at 850,553 bp of the public *S. oleracea* reference genome v1, marker M6 comprises a SNP change from C to T at 894,117 bp of the public *S. oleracea* reference genome v1, marker M7 comprises a SNP change from G to A at 1,401,465 bp of the public *S. oleracea* reference genome v1, marker M8 comprises a SNP change from C to T at 1,467,108 bp of the public *S. oleracea* reference genome v1, and marker M9 comprises a SNP change from G to T at 1,774,344 bp of the public *S. oleracea* reference genome v1. The public genome of spinach is available at, for example, SpinachBase (spinachbase.org), and one skilled in the art would understand how to locate the markers or any version (or later version) of the public genome using the marker sequences disclosed in the instant application.

In certain embodiments, the present disclosure provides methods of producing or selecting a spinach plant exhibiting resistance to downy mildew comprising: a) crossing a spinach plant provided herein with itself (also referred to as selfing) or with a second spinach plant of a different genotype to produce one or more progeny plants; and b) selecting a progeny plant comprising a downy mildew resistance allele. In some embodiments, methods of the present disclosure comprise selecting a progeny plant by detecting nucleic acids comprising marker M1 (SEQ ID NO: 1), marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), marker M8 (SEQ ID NO:8), or marker M9 (SEQ ID NO:9) on chromosome 3.

Because genetically diverse plant lines can be difficult to cross, the introgression of downy mildew resistance loci and/or alleles into cultivated lines using conventional breeding methods could require prohibitively large segregating populations for progeny screens with an uncertain outcome. Marker-assisted selection (MAS) is therefore essential for the effective introgression of loci that confer resistance to downy mildew into elite cultivars. For the first time, the present disclosure enables effective MAS by providing improved and validated markers for detecting genotypes associated with downy mildew resistance without the need to grow large populations of plants to maturity in order to observe the phenotype.

### I. Genomic Region, QTL, Polymorphic Nucleic Acids, and Alleles Associated With Downy Mildew Resistance

The present disclosure provides novel introgressions of one or more loci associated with resistance to downy mildew in spinach, together with polymorphic nucleic acids and linked markers for tracking the introgressions during plant breeding. The seed deposited under NCMA Accession No. 202210003 may be used as a source for the recombinant chromosomal segment on chromosome 3 that is described herein.

Using the improved genetic markers and assays described in the present disclosure, a novel chromosomal region was identified that confers resistance to downy mildew to a spinach plant. In certain embodiments, the present disclosure provides spinach plants comprising donor DNA between marker M1 (SEQ ID NO:1) and marker M9 (SEQ ID NO:9) on chromosome 3.

### II. Introgression of a Genomic Locus Associated with Resistance to Downy Mildew in Spinach

Marker-assisted introgression involves the transfer of a chromosomal region defined by one or more markers from a first genetic background to a second. Offspring of a cross that contain the introgressed genomic region can be identified by the combination of markers characteristic of the desired introgressed genomic region from a first genetic background and both linked and unlinked markers characteristic of the second genetic background.

The present disclosure provides novel accurate markers for identifying and tracking introgression of one or more of the genomic regions disclosed herein from a downy mildew resistant plant into a cultivated line. The present disclosure further provides markers for identifying and tracking the novel introgressions disclosed herein during plant breeding, including the markers set forth in Table 1.

Markers within or linked to any of the genomic intervals of the present disclosure may be useful in a variety of breeding efforts that include introgression of genomic regions associated with downy mildew resistance into a desired genetic background. For example, a marker within 40 cM, 20 cM, 15 cM, 10 cM, 5cM, 2 cM, or 1 cM of a marker associated with downy mildew resistance described herein can be used for marker-assisted introgression of genomic regions associated with a downy mildew resistant phenotype.

Markers that are linked and either immediately adjacent or adjacent to the identified downy mildew resistance QTL that permit introgression of the locus in the absence of extraneous linked DNA from the source germplasm containing the QTL are provided herewith. Those of skill in the art will appreciate that when seeking to introgress a smaller genomic region comprising a QTL associated with resistance to downy mildew described herein, that any of the telomere proximal or centromere proximal markers that are immediately adjacent to a larger genomic region comprising the QTL can be used to introgress that smaller genomic region.

Spinach plants or germplasm comprising an introgressed region that is associated with resistance to downy mildew wherein at least 10%, 25%, 50%, 75%, 90%, or 99% of the remaining genomic sequences carry markers characteristic of plant or germplasm that otherwise or ordinarily comprise a genomic region associated with another phenotype, are thus provided in specific embodiments. Furthermore, spinach plants comprising an introgressed region where closely linked regions adjacent and/or immediately adjacent to the genomic regions, QTL, and markers provided herewith that comprise genomic sequences carrying markers characteristic of spinach plants or germplasm that otherwise or ordinarily comprise a genomic region associated with the phenotype are also provided.

### III. Development of Spinach Plants with Resistance to Downy Mildew

For most breeding objectives, commercial breeders may work within germplasm that is often referred to as the "cultivated" or "elite." This germplasm is easier to use in plant breeding because it generally performs well when evaluated for horticultural performance. The performance advantage a cultivated variety provides is sometimes offset by a lack of allelic diversity. Breeders generally accept this tradeoff because progress is faster when working with cultivated material than when breeding with genetically diverse sources.

In contrast, when cultivated germplasm is crossed with non-cultivated germplasm, a breeder can gain access to novel alleles from the non-cultivated type. However, this approach presents significant difficulties due to fertility problems associated with crosses between diverse lines, and negative linkage drag from the non-cultivated parent. In spinach plants, non-cultivated types such as *S. tetrandra* can provide alleles associated with disease resistance. However, these non-cultivated types may have poor horticultural qualities.

The process of introgressing desirable resistance genes from non-cultivated lines into elite cultivated lines while avoiding problems with genetically linked deleterious loci or low heritability is a long and often arduous process. In deploying loci derived from wild relatives it is often desirable to introduce a minimal or truncated introgression that provides the desired trait but lacks detrimental effects. To aid introgression reliable marker assays are preferable to phenotypic screens. Success is furthered by simplifying genetics for key attributes to allow focus on genetic gain for quantitative traits such as downy mildew resistance. Moreover, the process of introgressing genomic regions from non-cultivated lines can be greatly facilitated by the availability of accurate markers for MAS.

One of skill in the art would therefore understand that the loci, polymorphisms, and markers provided by the present disclosure allow the tracking and introduction of any of the genomic regions identified herein into any genetic background. In addition, the genomic regions associated with downy mildew resistance disclosed herein can be introgressed from one genotype to another and tracked using MAS. Thus, the inventors' discovery of accurate markers associated with downy mildew resistance will facilitate the development of spinach plants having beneficial phenotypes. For example, seed can be genotyped using the markers of the present disclosure to select for plants comprising desired genomic regions associated with downy mildew resistance. Moreover, MAS allows identification of plants that are homozygous or heterozygous for a desired introgression.

Inter-species crosses can also result in suppressed recombination and plants with low fertility or fecundity. For example, suppressed recombination has been observed for the tomato nematode resistance gene *Mi,* the *Mla* and *Mlg* genes in barley, the *Yr17* and *Lr20* genes in wheat, the *Run1* gene in grapevine, and the *Rma* gene in peanut. Meiotic recombination is essential for classical breeding because it enables the transfer of favorable loci across genetic backgrounds, the removal of deleterious genomic fragments, and pyramiding traits that are genetically tightly linked. Therefore, suppressed recombination forces breeders to enlarge segregating populations for progeny screens in order to arrive at the desired genetic combination.

Phenotypic evaluation of large populations is time-consuming, resource-intensive and not reproducible in every environment. Marker-assisted selection offers a feasible alternative. Molecular assays designed to detect unique polymorphisms, such as SNPs, are versatile. However, they may fail to discriminate loci within and among spinach species in a single assay. Structural rearrangements of chromosomes such as deletions impair hybridization and extension of synthetically labeled oligonucleotides. In the case of duplication events, multiple copies are amplified in a single reaction without distinction. The development and validation of accurate and highly predictive markers are therefore essential for successful MAS breeding programs.

### IV. Molecular Assisted Breeding Techniques

Genetic markers that can be used in the practice of the present disclosure include, but are not limited to, restriction fragment length polymorphisms (RFLPs), amplified fragment length polymorphisms (AFLPs), simple sequence repeats (SSRs), simple sequence length polymorphisms (SSLPs), single nucleotide polymorphisms (SNPs), insertion/deletion polymorphisms (Indels), variable number tandem repeats (VNTRs), and random amplified polymorphic DNA (RAPD), isozymes, and other markers known to those skilled in the art. Marker discovery and development in crop plants provides the initial framework for applications to marker-assisted breeding activities (U.S. Patent Pub. Nos.: 2005/0204780, 2005/0216545, 2005/0218305, and 2006/00504538). The resulting "genetic map" is the representation of the relative position of characterized loci (polymorphic nucleic acid markers or any other locus for which loci can be identified) to each other.

Polymorphisms comprising as little as a single nucleotide change can be assayed in a number of ways. For example, detection can be made by electrophoretic techniques including a single strand conformational polymorphism (Orita et al., Genomics 8(2):271-278, 1998), denaturing gradient gel electrophoresis (Myers (1985) EP 0273085), or cleavage fragment length polymorphisms (Life Technologies, Inc., Gaithersburg, MD), but the widespread availability of DNA sequencing often makes it easier to simply sequence amplified products directly. Once the polymorphic sequence difference is known, rapid assays can be designed for progeny testing, typically involving some version of PCR amplification of specific loci (PASA; Sommer et al., Biotechniques 12(1):82-87, 1992), or PCR amplification of multiple specific loci (PAMSA; Dutton and Sommer, Biotechniques, 11(6):700-7002, 1991).

Polymorphic markers serve as useful tools for assaying plants for determining the degree of identity of lines or varieties (U.S. Patent No. 6,207,367). These markers form the basis for determining associations with phenotypes and can be used to drive genetic gain. In certain embodiments of the methods of the present disclosure, polymorphic nucleic acids can be used to detect in a spinach plant a genotype associated with downy mildew resistance, identify a spinach plant with a genotype associated with downy mildew resistance, and to select a spinach plant with a genotype associated with downy mildew resistance. In certain embodiments of methods of the present disclosure, polymorphic nucleic acids can be used to produce a spinach plant that comprises in its genome an introgressed locus associated with downy mildew resistance. In certain embodiments of the present disclosure, polymorphic nucleic acids can be used to breed progeny spinach plants comprising a locus or loci associated with downy mildew resistance.

Genetic markers may include "dominant" or "codominant" markers. "Codominant" markers reveal the presence of two or more loci (two per diploid individual). "Dominant" markers reveal the presence of only a single locus. Markers are preferably inherited in codominant fashion so that the presence of both loci at a diploid locus, or multiple loci in triploid or tetraploid loci, are readily detectable, and they are free of environmental variation, i.e., their heritability is 1. A marker genotype typically comprises two marker loci at each locus in a diploid organism. The marker allelic composition of each locus can be either homozygous or heterozygous. Homozygosity is a condition where both loci at a locus are characterized by the same nucleotide sequence. Heterozygosity refers to a condition where the two loci at a locus are different.

Nucleic acid-based analyses for determining the presence or absence of the genetic polymorphism (i.e. for genotyping) can be used in breeding programs for identification, selection, introgression, and the like. A wide variety of genetic markers for the analysis of genetic polymorphisms are available and known to those of skill in the art. The analysis may be used to select for genes, portions of genes, QTL, loci, or genomic regions that comprise or are linked to a genetic marker that is linked to or associated with downy mildew resistance in spinach plants.

As used herein, nucleic acid analysis methods include, but are not limited to, PCR-based detection methods (for example, TaqMan assays), microarray methods, mass spectrometry-based methods and/or nucleic acid sequencing methods, including whole genome sequencing. In certain embodiments, the detection of polymorphic sites in a sample of DNA, RNA, or cDNA may be facilitated through the use of nucleic acid amplification methods. Such methods specifically increase the concentration of polynucleotides that span the polymorphic site, or include that site and sequences located either distal or proximal to it. Such amplified molecules can be readily detected by gel electrophoresis, fluorescence detection methods, or other means.

One method of achieving such amplification employs the polymerase chain reaction (PCR) (Mullis et al., Cold Spring Harbor Symp. Quant. Biol. 51:263-273, 1986; European Patent No. 50,424; European Patent No. 84,796; European Patent No. 258,017; European Patent No. 237,362; European Patent No. 201,184; U.S. Patent No. 4,683,202; U.S. Patent No. 4,582,788; and U.S. Patent No. 4,683,194), using primer pairs that are capable of hybridizing to the proximal sequences that define a polymorphism in its double-stranded form. Methods for typing DNA based on mass spectrometry can also be used. Such methods are disclosed in U.S. Patent Nos. 6,613,509 and 6,503,710, and references found therein.

Polymorphisms in DNA sequences can be detected or typed by a variety of effective methods well known in the art including, but not limited to, those disclosed in U.S. Patent Nos. 5,468,613, 5,217,863; 5,210,015; 5,876,930; 6,030,787; 6,004,744; 6,013,431; 5,595,890; 5,762,876; 5,945,283; 5,468,613; 6,090,558; 5,800,944; 5,616,464; 7,312,039; 7,238,476; 7,297,485; 7,282,355; 7,270,981; and 7,250,252, all of which are incorporated herein by reference in their entirety. However, the compositions and methods of the present disclosure can be used in conjunction with any polymorphism typing method to detect polymorphisms in genomic DNA samples. These genomic DNA samples used include but are not limited to, genomic DNA isolated directly from a plant, cloned genomic DNA, or amplified genomic DNA.

For instance, polymorphisms in DNA sequences can be detected by hybridization to locus-specific oligonucleotide (ASO) probes as disclosed in U.S. Patent Nos. 5,468,613 and 5,217,863. U.S. Patent No. 5,468,613 discloses locus specific oligonucleotide hybridizations where single or multiple nucleotide variations in nucleic acid sequence can be detected in nucleic acids by a process in which the sequence containing the nucleotide variation is amplified, spotted on a membrane and treated with a labeled sequence-specific oligonucleotide probe.

Target nucleic acid sequence can also be detected by probe ligation methods, for example as disclosed in U.S. Patent No. 5,800,944 where sequence of interest is amplified and hybridized to probes followed by ligation to detect a labeled part of the probe.

Microarrays can also be used for polymorphism detection, wherein oligonucleotide probe sets are assembled in an overlapping fashion to represent a single sequence such that a difference in the target sequence at one point would result in partial probe hybridization (Borevitz et al., Genome Res. 13:513-523, 2003; Cui et al., Bioinformatics 21:3852-3858, 2005). On any one microarray, it is expected there will be a plurality of target sequences, which may represent genes and/or noncoding regions wherein each target sequence is represented by a series of overlapping oligonucleotides, rather than by a single probe. This platform provides for high throughput screening of a plurality of polymorphisms. Typing of target sequences by microarray-based methods is described in US Patents 6,799,122; 6,913,879; and 6,996,476.

Other methods for detecting SNPs and Indels include single base extension (SBE) methods. Examples of SBE methods include, but are not limited, to those disclosed in U.S. Patent Nos. 6,004,744; 6,013,431; 5,595,890; 5,762,876; and 5,945,283.

In another method for detecting polymorphisms, SNPs and Indels can be detected by methods disclosed in U.S. Patent Nos. 5,210,015; 5,876,930; and 6,030,787 in which an oligonucleotide probe having a 5' fluorescent reporter dye and a 3' quencher dye covalently linked to the 5' and 3' ends of the probe. When the probe is intact, the proximity of the reporter dye to the quencher dye results in the suppression of the reporter dye fluorescence, e.g. by Forster-type energy transfer. During PCR, forward and reverse primers hybridize to a specific sequence of the target DNA flanking a polymorphism while the hybridization probe hybridizes to polymorphism-containing sequence within the amplified PCR product. In the subsequent PCR cycle DNA polymerase with 5' → 3' exonuclease activity cleaves the probe and separates the reporter dye from the quencher dye resulting in increased fluorescence of the reporter.

In another embodiment, a locus or loci of interest can be directly sequenced using nucleic acid sequencing technologies. Methods for nucleic acid sequencing are known in the art and include technologies provided by 454 Life Sciences (Branford, CT), Agencourt Bioscience (Beverly, MA), Applied Biosystems (Foster City, CA), LI-COR Biosciences (Lincoln, NE), NimbleGen Systems (Madison, WI), Illumina (San Diego, CA), and VisiGen Biotechnologies (Houston, TX). Such nucleic acid sequencing technologies comprise formats such as parallel bead arrays, sequencing by ligation, capillary electrophoresis, electronic microchips, "biochips," microarrays, parallel microchips, and single-molecule arrays.

Various genetic engineering or gene editing technologies have been developed and may be used by those of skill in the art to introduce traits in plants. In certain aspects of the present disclosure, traits are introduced into spinach plants via altering or introducing a single genetic locus, site-specific modification, or transgene into the genome of a variety or progenitor thereof. Methods of genetic engineering to modify, delete, or insert genes and polynucleotides into the genomic DNA of plants are well-known in the art.

In specific embodiments of the present disclosure, improved spinach lines can be created through the site-specific modification of a plant genome. In certain embodiments, a site-specific modification may be referred to as a gene edit. Methods of genetic engineering include, for example, utilizing sequence-specific nucleases such as zinc-finger nucleases (see, for example, U.S. Pat. Appl. Pub. No. 2011/0203012); engineered or native meganucleases; TALE-endonucleases (see, for example, U.S. Pat. Nos. 8,586,363 and 9,181,535); and RNA-guided endonucleases, such as those of the CRISPR/Cas systems (see, for example, U.S. Pat. Nos. 8,697,359; 8,771,945; and 10,266,850). One embodiment of the present disclosure thus relates to utilizing a nuclease or any associated protein to carry out genome modification. This nuclease could be provided heterologously within donor template DNA for templated-genomic editing or in a separate molecule or vector. A recombinant DNA construct may also comprise a sequence encoding one or more guide RNAs to direct the nuclease to the site within the plant genome to be modified. Further methods for altering or introducing a single genetic locus include, for example, utilizing single-stranded oligonucleotides to introduce base pair modifications in a plant genome (see, for example, Sauer et al., Plant Physiol, 170(4):1917-1928, 2016).

Methods for site-directed alteration or introduction of a single genetic locus are well-known in the art and include those that utilize sequence-specific nucleases, such as the aforementioned, or complexes of proteins and guide-RNA that cut genomic DNA to produce a double-strand break (DSB) or nick at a genetic locus. In certain embodiments, methods for producing a site-directed alteration may be referred to as gene editing. As is well-understood in the art, during the process of repairing the DSB or nick introduced by the nuclease enzyme, a donor template, transgene, or expression cassette polynucleotide may become integrated into the genome at the site of the DSB or nick. The presence of homology arms in the DNA to be integrated may promote the adoption and targeting of the insertion sequence into the plant genome during the repair process through homologous recombination or non-homologous end joining (NHEJ).

In another embodiment of the present disclosure, genetic transformation may be used to insert a selected transgene into a plant of the present disclosure or may, alternatively, be used for the preparation of transgenes which can be introduced by backcrossing. Methods for the transformation of plants that are well-known to those of skill in the art and applicable to many crop species include, but are not limited to, electroporation, microprojectile bombardment, *Agrobacterium*-mediated transformation, and direct DNA uptake by protoplasts.

To effect transformation by electroporation, one may employ either friable tissues, such as a suspension culture of cells or embryogenic callus or alternatively one may transform immature embryos or other organized tissue directly. In this technique, one would partially degrade the cell walls of the chosen cells by exposing them to pectin-degrading enzymes (pectolyases) or mechanically wound tissues in a controlled manner.

An efficient method for delivering transforming DNA segments to plant cells is microprojectile bombardment. In this method, particles are coated with nucleic acids and delivered into cells by a propelling force. Exemplary particles include those comprised of tungsten, platinum, and preferably, gold. For the bombardment, cells in suspension are concentrated on filters or solid culture medium. Alternatively, immature embryos or other target cells may be arranged on solid culture medium. The cells to be bombarded are positioned at an appropriate distance below the microprojectile stopping plate.

An illustrative embodiment of a method for delivering DNA into plant cells by acceleration is the Biolistics Particle Delivery System, which can be used to propel particles coated with DNA or cells through a screen, such as a stainless steel or Nytex screen, onto a surface covered with target cells. The screen disperses the particles so that they are not delivered to the recipient cells in large aggregates. Microprojectile bombardment techniques are widely applicable and may be used to transform virtually any plant species.

*Agrobacterium*-mediated transfer is another widely applicable system for introducing gene loci into plant cells. An advantage of the technique is that DNA can be introduced into whole plant tissues, thereby bypassing the need for regeneration of an intact plant from a protoplast. Modern *Agrobacterium* transformation vectors are capable of replication in *E. coli* as well as *Agrobacterium*, allowing for convenient manipulations (Klee et al., Nat. Biotechnol., 3(7):637-642, 1985). Moreover, recent technological advances in vectors for *Agrobacterium*-mediated gene transfer have improved the arrangement of genes and restriction sites in the vectors to facilitate the construction of vectors capable of expressing various polypeptide coding genes. The vectors described have convenient multi-linker regions flanked by a promoter and a polyadenylation site for direct expression of inserted polypeptide coding genes. Additionally, *Agrobacterium* containing both armed and disarmed Ti genes can be used for transformation.

In those plant strains where *Agrobacterium*-mediated transformation is efficient, it is the method of choice because of the facile and defined nature of the gene locus transfer. The use of *Agrobacterium*-mediated plant integrating vectors to introduce DNA into plant cells is well known in the art (Fraley et al., Nat. Biotechnol., 3:629-635, 1985; U.S. Patent No. 5,563,055).

Transformation of plant protoplasts also can be achieved using methods based on calcium phosphate precipitation, polyethylene glycol treatment, electroporation, and combinations of these treatments (see, for example, Potrykus et al., Mol. Gen. Genet., 199:183-188, 1985; Omirulleh et al., Plant Mol. Biol., 21(3):415-428, 1993; Fromm et al., Nature, 312:791-793, 1986; Uchimiya et al., Mol. Gen. Genet., 204:204, 1986; Marcotte et al., Nature, 335:454, 1988). Transformation of plants and expression of foreign genetic elements is exemplified in Choi et al. (Plant Cell Rep., 13:344-348, 1994), and Ellul et al. (Theor. Appl. Genet., 107:462-469, 2003).

### V. Edible Compositions

As used herein, "edible composition" refers to a composition which may be ingested by a mammal such as a food or feed product or a pharmaceutical composition. As used herein "food" and "feed products" refer to substances that can be used or prepared for use as food for an animal or human and include substances that may be used in the preparation of food or as food additives. Typical food or feed products include but are not limited to soups, juices, smoothies, spreads, yogurts, sauces, gravies, quiches, pies, prepared vegetable products, such as a vegetable bake, and blended vegetable products. Furthermore, edible compositions described herein may also be ingested as an additive or supplement. These can be formulated together with a nutritional substances, such as various vitamins and minerals, and incorporated into substantially liquid compositions, substantially solid compositions, or gelatins. Edible compositions as described herein may, in some embodiments, be in powder form.

As used herein "pharmaceutical composition" refers to an composition comprising a pharmaceutically acceptable carrier. In a specific embodiment, the term "pharmaceutically acceptable" refers to a carrier suitable for administration to an animal or human. The edible compositions of the present disclosure may for example comprise any pharmaceutically acceptable carrier selected with regard with the intended route of administration and standard pharmaceutical practice. The edible compositions of the present disclosure may for example be administered as a tablet, a capsule, an elixir, a solution, or a suspension.

The edible compositions described here may be produced using any method known in the art, non-limiting examples of which include mixing, blending, freezing, heating, cooling, lyophilizing, pasteurizing, and cooking using any suitable method.

The spinach of the present invention can be used in any product incorporating spinach including but not limited to the use of the spinach, a part of the spinach, an extract or derivative of the spinach, or a cell of the spinach. The spinach can be used as an ingredient, component, formulation, extract, or derivative. The spinach can be present as identifiable parts or non-identifiable parts. The spinach can be fresh, frozen, or dehydrated. The spinach can be used in any food, beverage, extract, supplement, nutraceutical, or the like for all purposes including but not limited to human or animal food, feed, or supplements. In particular, the spinach can be used as a fresh or frozen intact spinach product or intact or processed in a soup, ready to eat meal, snack bar, drink, smoothie, shake, tablet, capsule, injectable, vitamin, or other similar products.

### VI. Definitions

The following definitions are provided to better define the present disclosure and to guide those of ordinary skill in the art in the practice of the present disclosure. Unless otherwise noted, terms are to be understood according to conventional usage by those of ordinary skill in the relevant art.

The term "about" is used to indicate that a value includes the standard deviation of error for the device or method being employed to determine the value. The use of the term "or" in the claims is used to mean "and/or" unless explicitly indicated to refer to alternatives only or the alternatives are mutually exclusive, although the disclosure supports a definition that refers to only alternatives and to "and/or." When used in conjunction with the word "comprising" or other open language in the claims, the words "a" and "an" denote "one or more," unless specifically noted. The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and also covers other unlisted steps. Similarly, any plant that "comprises," "has" or "includes" one or more traits is not limited to possessing only those one or more traits and covers other unlisted traits

As used herein, the term "plant" includes the seed (from which the plant can be grown), the whole plant or any plant parts, such as plant organs (e.g., harvested or non-harvested leaves, etc.), plant cells, plant protoplasts, plant cell cultures, or tissue cultures from which whole plants can be regenerated, propagating or non-propagating plant cells, plants cells which are not in tissue culture (but which are, for example, *in vivo* in a plant or plant part), plant callus, plant cell clumps, plant transplants, seedlings, plant cells that are intact in plants, plant clones or micro-propagations, or parts of plants (e.g., harvested tissues or organs), such as plant cuttings, vegetative propagations, embryos, pollen, ovules, flowers, leaves, seeds (produced on the plant after self-fertilization or cross-fertilization), clonally propagated plants, roots, stems, shoots, stalks, root tips, grafts, parts of any of these and the like, or derivatives thereof, preferably having the same genetic make-up (or very similar genetic make-up) as the plant from which it is obtained. Also any developmental stage is included, such as seedlings, cuttings prior or after rooting, mature and/or immature plants or mature and/or immature leaves. When "seeds of a plant" are referred to, these either refer to seeds from which the plant can be grown or to seeds produced on the plant, after self-fertilization or cross-fertilization.

As used herein, the term "population" means a genetically heterogeneous collection of plants that share a common parental derivation.

As used herein, the term "plant line" is, for example, a breeding line which can be used to develop one or more varieties. "Inbred line" or "inbred parent" is a line which has been developed by selfing for several generations and which can be used as a parent to produce an F₁ hybrid variety. "Hybrid" refers to the seeds harvested from crossing one plant line or variety with another plant line or variety, and the plants or plant parts grown from said seeds.

As used herein, the term "F₁ hybrid" plant (or F₁ hybrid seed) is the generation obtained from crossing two non-isogenic inbred parent lines. Thus, F₁ hybrid seeds are seeds from which F₁ hybrid plants grow.

As used herein, the term "interspecific hybrid" refers to a hybrid produced from crossing a plant of one species, e.g., *S*. *oleracea,* with a plant of another species, e.g., *S. tetrandra* or *S*. *turkestanica.*

As used herein, the terms "progeny," "progenies," or "descendants" as used herein, refer to offspring, or the first and all further descendants derived from (or obtainable from) a plant. Progeny may be derived by regeneration of cell culture or tissue culture, or parts of a plant, or selfing of a plant, or by producing seeds of a plant. In further embodiments, progeny may also encompass spinach plants derived from crossing of at least one spinach plant with another spinach plant of the same or another variety or (breeding) line, and/or backcrossing, and/or inserting of a locus into a plant and/or mutation. A progeny is, e.g., a first generation progeny, i.e., the progeny is directly derived from, obtained from, obtainable from or derivable from the parent plant by, e.g., traditional breeding methods (selfing and/or crossing) or regeneration. However, the term "progeny" generally encompasses further generations such as second, third, fourth, fifth, sixth, seventh or more generations, i.e., generations of plants which are derived from, obtained from, obtainable from or derivable from the former generation by, e.g., traditional breeding methods, regeneration or genetic transformation techniques. For example, a second generation progeny can be produced from a first generation progeny by any of the methods mentioned above. Also double haploid plants are progeny.

As used herein, the term "tissue culture" or "cell culture" refers to an *in vitro* composition comprising isolated cells of the same or a different type or a collection of such cells organized into plant tissue. Tissue cultures and cell cultures of spinach, and regeneration of spinach plants therefrom, is well known in the art and widely published.

As used herein, the term "regeneration" refers to the development of a plant from *in vitro* cell culture or tissue culture or vegetative propagation.

As used herein, the term "vegetative propagation," "vegetative reproduction," and "clonal propagation" are used interchangeably herein and refer to the method of taking part of a plant and allowing that plant part to form at least roots where plant part is, e.g., defined as or derived from (e.g., by cutting off) leaf, pollen, embryo, cotyledon, hypocotyl, cells, protoplasts, meristematic cell, root, root tip, pistil, anther, flower, shoot tip, shoot, stem, fruit, and petiole. When a whole plant is regenerated by vegetative propagation, it is also referred to as a "vegetative propagation" or a "vegetatively propagated plant."

As used herein, the term "harvested plant material" refers to plant parts (e.g., leaves detached from the whole plant) which have been collected for further storage and/or further use. As used herein, the term "harvested seeds" refers to seeds harvested from a line or variety, e.g., produced after self-fertilization or cross-fertilization and collected. As used herein, the term "harvested leaves" refers to spinach leaves, *i.e.*, the plant without the root system, for example substantially all (harvested) leaves.

As used herein, the terms "variety" and "cultivar" mean a group of similar plants that by their genetic pedigrees and performance can be identified from other varieties within the same species.

As used herein, "elite" or "cultivated" variety means any variety that has resulted from breeding and selection for superior agronomic performance. An "elite plant" refers to a plant belonging to an elite variety. Numerous elite varieties are available and known to those of skill in the art of spinach breeding. An "elite population" is an assortment of elite individuals or varieties that can be used to represent the state of the art in terms of agronomically superior genotypes of a given crop species, such as spinach. Similarly, an "elite germplasm" or elite strain of germplasm is an agronomically superior germplasm. An "elite spinach" or "cultivated spinach" cultivar/variety refers herein to plants of the species *Spinacia oleracea* (or seeds from which the plants can be grown), and parts of such plants, bred by humans for food and having good agronomic characteristics. This includes any cultivated spinach, such as breeding lines (e.g. backcross lines, inbred lines), cultivars, and varieties (open-pollinated or hybrids). This includes any type of spinach, such as savoy, flat- or smooth-leaf spinach, or semi-savoy types. Wild spinach (i.e. not cultivated spinach) or wild relatives of spinach, such as *Spinacia tetrandra* and *Spinacia turkestanica*, are not encompassed by this definition.

As used herein, an "allele" refers to one of two or more alternative forms of a genomic sequence at a given locus on a chromosome. All alleles at a specific locus relate to one trait or characteristic. In a diploid cell of an organism, alleles of a given gene are located at a specific location, or locus on a chromosome. One allele is present on each chromosome of the pair of homologous chromosomes. A diploid plant species may comprise a large number of different alleles at a particular locus. These may be identical alleles of the gene (homozygous) or two different alleles (heterozygous).

As used herein, "downy mildew" refers to a disease of plants caused by pathogenic oomycetes of the *Peronosporaceae* family. Downy mildew on spinach is caused by *Peronospora effusa. Peronospora effusa* is a distinct *Peronospora* species whose host range is believed to be restricted to spinach.

As used herein, "race" refers to an officially designated strain of *Peronospora effusa* that can cause downy mildew. As used herein, "isolate" refers to a newly occurring strain of *Peronospora effusa* that can cause downy mildew, and has not yet been officially named. Races are classified based on their reaction pattern when inoculated on the differential hosts of spinach.

As used herein, "differentials," "differential hosts," or "differential lines," refer to the standardized set of plant cultivars used to identify and distinguish different biotypes, pathotypes, races, or strains within a (sub)species. For downy mildew in spinach, the differential set has been determined by the International Working Group on *Peronospora* (IWGP). This can be obtained from the Naktuinbouw, P.O. Box 40, 2370 AA Roelofarendsveen, The Netherlands, or via references provided by the ISF (International Seed Federation).

As used herein, the term "locus" or "loci" refers to a specific place or places or a site on a chromosome where, for example, a gene or genetic marker is found. A "quantitative trait locus (QTL)" is a chromosomal location that encodes for at least a first allele that affects the expressivity of a phenotype.

As used herein, an "introgression fragment" or "introgression segment" or "introgression region" refers to a chromosome fragment (or chromosome part or region) which has been introduced into another plant of the same or related species by crossing or traditional breeding techniques, such as backcrossing, i.e., the introgressed fragment is the result of breeding methods referred to by the verb "to introgress" (such as backcrossing). In spinach, wild spinach, or wild relatives of spinach are used to introgress fragments of the wild genome into the genome of cultivated spinach. Such a spinach plant thus has a "genome of *Spinacia oleracea*" but comprises in the genome a fragment of a wild spinach or spinach relative, i.e., an introgression fragment of a donor plant. It is understood that the term "introgression fragment" never includes a whole chromosome, but only a part of a chromosome.

A genetic element, a locus, an introgression fragment or a gene or allele conferring a trait (such as downy mildew resistance) is said to be "obtainable from" or can be "obtained from" or "derivable from" or can be "derived from" or "as present in" or "as found in" a plant or seed if it can be transferred from the plant or seed in which it is present into another plant or seed in which it is not present (such as a line or variety) using traditional breeding techniques without resulting in a phenotypic change of the recipient plant apart from the addition of the trait conferred by the genetic element, locus, introgression fragment, gene, or allele. The terms are used interchangeably and the genetic element, locus, introgression fragment, gene, or allele can thus be transferred into any other genetic background lacking the trait. Not only seeds deposited and comprising the genetic element, locus, introgression fragment, gene, or allele can be used, but also progeny/descendants from such seeds which have been selected to retain the genetic element, locus, introgression fragment, gene, or allele, can be used and are encompassed herein, such as commercial varieties developed from the deposited seeds or from descendants thereof. Whether a plant comprises the same genetic element, locus, introgression fragment, gene, or allele as obtainable from the deposited seeds can be determined by the skilled person using one or more techniques known in the art, such as phenotypic assays, whole genome sequencing, molecular marker analysis, trait mapping, chromosome painting, allelism tests, and the like.

As used herein, a "marker" refers to detectable characteristic that can be used to discriminate between organisms. Examples of such characteristics include, but are not limited to, genetic markers, biochemical markers, metabolites, morphological characteristics, and agronomic characteristics.

As used herein, a "molecular marker" is a piece of DNA associated with a certain genomic or chromosomal location or single nucleotide polymorphism (SNP), which is found on the chromosome close to the gene of interest. Molecular markers can be used to identify a particular sequence of DNA, or a certain location in a genome or on a chromosome, or to identify an introgression fragment. When reference is made herein to one or more molecular markers being "detectable" by a molecular marker assay, this means of course that the plant or plant part comprises the one or more markers in its genome, as the marker would otherwise not be detectable.

As used herein, "flanking markers" or "bordering markers" are molecular markers located on the chromosome on either side of an allele or gene of interest, i.e., one marker on the right side of the allele or gene and one marker on the left side of the allele or gene.

As used herein, "closely linked marker" is a marker which is physically close enough to an allele or gene to co-segregate with the allele or gene at a high frequency, *i.e.*, the chance of recombination taking place between the allele or gene and the marker is so small that the marker can be used to reliably select for the presence of the allele or gene in a breeding program (marker-assisted selection).

As used herein, "marker-assisted selection" or "MAS" refers to a process of using the presence of molecular markers, which are genetically and physically linked to a particular locus or to a particular chromosomal region (e.g., introgression fragment), to select plants (e.g., progeny) for the presence of the specific locus or region (e.g., introgression fragment).

As used herein, "marker assay" or "genotyping assay" refers to an assay which can be used to determine the marker genotype, e.g., the SNP genotype. For example, SNP markers can be detected using a KASP-assay or other assays known to the skilled person.

As used herein, the term "phenotype" refers to the detectable characteristics of a cell or organism that can be influenced by gene expression.

As used herein, the term "genotype" refers to the specific allelic makeup of a plant.

As used herein, a "gene" refers to a nucleic acid sequence forming a genetic and functional unit and coding for one or more sequence-related RNA and/or polypeptide molecules. A gene generally contains a coding region operably linked to appropriate regulatory sequences that regulate the expression of a gene product (e.g., a polypeptide or a functional RNA). A gene can have various sequence elements, including, but not limited to, a promoter, an untranslated region (UTR), exons, introns, and other upstream or downstream regulatory sequences.

As used herein, the term "physical distance" referring to a region between loci (e.g., between molecular markers and/or between phenotypic markers) on the same chromosome is the actual physical distance expressed in base pairs (bp), kilobase pairs (kb), or megabase pairs (Mb).

As used herein, the term "genetic distance" between loci (e.g., between molecular markers and/or between phenotypic markers) on the same chromosome is measured by frequency of crossing-over, or recombination frequency (RF) and is indicated in centimorgans (cM). One cM corresponds to a recombination frequency of 1%. If no recombinants can be found, the RF is zero and the loci are either extremely close together physically or they are identical. The further apart two loci are, the higher the RF.

As used herein, the term "introgressed," when used in reference to a genetic locus, refers to a genetic locus that has been introduced into a new genetic background, such as through backcrossing. Introgression of a genetic locus can thus be achieved through plant breeding methods and/or by molecular genetic methods. Such molecular genetic methods include, but are not limited to, various plant transformation techniques and/or methods that provide for homologous recombination, non-homologous recombination, site-specific recombination, and/or genomic modifications that provide for locus substitution or locus conversion.

As used herein, the terms "recombinant" or "recombined" in the context of a chromosomal segment refer to recombinant DNA sequences comprising one or more genetic loci in a configuration in which they are not found in nature, for example as a result of a recombination event between homologous chromosomes during meiosis.

As used herein, the term "linked," when used in the context of nucleic acid markers and/or genomic regions, refers to markers and/or genomic regions that are located on the same linkage group or chromosome such that they tend to segregate together at meiosis.

As used herein, "tolerance locus" means a locus associated with tolerance or resistance to disease. For instance, a tolerance locus according to the present disclosure may, in one embodiment, control tolerance or susceptibility to *Peronospora effusa.*

As used herein, "tolerance" or "improved tolerance" in a plant refers to the ability of the plant to perform well, for example by maintaining yield, under disease conditions or upon pest infestations. Tolerance may also refer to the ability of a plant to maintain a plant vigor phenotype under disease conditions or under pest infestations. Tolerance is a relative term, indicating that a "tolerant" plant is more able to maintain performance compared to a different (less tolerant) plant (e.g. a different plant variety) grown in similar disease conditions or under similar pest pressure. One of skill will appreciate that plant tolerance to disease or pest conditions varies widely and can represent a spectrum of more-tolerant or less-tolerant phenotypes. However, by simple observation, one of skill can generally determine the relative tolerance of different plants, plant varieties, or plant families under disease or pest conditions, and furthermore, will also recognize the phenotypic gradations of "tolerance."

As used herein "resistance" or "improved resistance" in a plant to disease or pest conditions is an indication that the plant is more able to reduce disease or pest burden than a non-resistant or less resistant plant. Resistance is a relative term, indicating that a "resistant" plant is more able to reduce disease burden or pest burden compared to a different (less resistant) plant (e.g., a different plant variety) grown in similar disease conditions or pest pressure. One of skill will appreciate that plant resistance to disease conditions or pest infestation varies widely and can represent a spectrum of more-resistant or less-resistant phenotypes. However, by simple observation, one of skill can generally determine the relative resistance of different plants, plant varieties, or plant families under disease conditions or pest pressure, and furthermore, will also recognize the phenotypic gradations of "resistant." Resistance can be non-specific or "broad-spectrum" or be race-specific.

As used herein, "broad-spectrum" resistance refers to resistance against more than one pathogen species or against most races or strains of the same species. "Species-nonspecific" broad-spectrum resistance refers to plant disease resistance against more than one pathogen species whereas "race-nonspecific" broad-spectrum resistance refers to plant disease resistance against multiple races or strains of the same pathogen species.

The terms "percent identity," "% identity," or "percent identical" as used herein in reference to two or more nucleotide or protein sequences is calculated by (i) comparing two optimally aligned sequences (nucleotide or protein) over a window of comparison, (ii) determining the number of positions at which the identical nucleic acid base (for nucleotide sequences) or amino acid residue (for proteins) occurs in both sequences to yield the number of matched positions, (iii) dividing the number of matched positions by the total number of positions in the window of comparison, and then (iv) multiplying this quotient by 100% to yield the percent identity. If the "percent identity" is being calculated in relation to a reference sequence without a particular comparison window being specified, then the percent identity is determined by dividing the number of matched positions over the region of alignment by the total length of the reference sequence. Accordingly, for purposes of the present application, when two sequences (query and subject) are optimally aligned (with allowance for gaps in their alignment), the "percent identity" for the query sequence is equal to the number of identical positions between the two sequences divided by the total number of positions in the query sequence over its length (or a comparison window), which is then multiplied by 100%. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity can be adjusted upwards to correct for the conservative nature of the substitution. Sequences that differ by such conservative substitutions are said to have "sequence similarity" or "similarity." Sequences having a percent identity to a base sequence may exhibit the activity of the base sequence.

As used herein, the term "denoting" when used in reference to a plant genotype refers to any method whereby a plant is indicated to have a certain genotype. This includes any means of identification of a plant having a certain genotype. Indication of a certain genotype may include, but is not limited to, any entry into any type of written or electronic medium or database whereby the plant's genotype is provided. Indications of a certain genotype may also include, but are not limited to, any method where a plant is physically marked or tagged. Illustrative examples of physical marking or tags useful in the present disclosure include, but are not limited to, a barcode, a radio-frequency identification (RFID), a label, or the like.

### VII. Deposit Information

A deposit of at least 625 seeds of spinach line MSA-S021-1336M was made with the Provasoli-Guillard National Center for Marine Algae and Microbiota (NCMA), 60 Bigelow Drive, East Boothbay, Maine, 04544 USA. The deposit is assigned NCMA Accession No. 202210003, and the date of deposit was June 10, 2022. Access to the deposit will be available during the pendency of the application to persons entitled thereto upon request. The deposit has been accepted under the Budapest Treaty and will be maintained in the NCMA Depository, which is a public depository, for a period of 30 years, or 5 years after the most recent request, or for the enforceable life of the patent, whichever is longer, and will be replaced if nonviable during that period. Applicant does not waive any infringement of their rights granted under this patent or any other form of variety protection, including the Plant Variety Protection Act (7 U.S.C. 2321 *et seq.*)*.*

### EMBODIMENTS

For further illustration, additional non-limiting embodiments of the present disclosure are set forth below.

Embodiment 1 is a *Spinacia oleracea* plant comprising a recombinant chromosomal segment from *Spinacia tetrandra* on chromosome 3, wherein said recombinant chromosomal segment comprises an allele that confers broad-spectrum resistance to *Peronospora effusa* relative to a plant lacking said recombinant chromosomal segment.

Embodiment 2 is the *Spinacia oleracea* plant of Embodiment 1, wherein said recombinant chromosomal segment comprises a marker locus selected from the group consisting of: marker M1 (SEQ ID NO:1), marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), marker M8 (SEQ ID NO:8), and marker M9 (SEQ ID NO:9).

Embodiment 3 is the *Spinacia oleracea* plant of Embodiment 1 or 2, wherein said recombinant chromosomal segment comprises a marker locus selected from the group consisting of: marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), and marker M8 (SEQ ID NO:8).

Embodiment 4 is the *Spinacia oleracea* plant of any one of the previous Embodiments, wherein the broad-spectrum resistance comprises resistance to at least *Peronospora effusa* races Pe:14, Pe:15, Pe:16, and Pe:17.

Embodiment 5 is the *Spinacia oleracea* plant of Embodiment 4, wherein the broad-spectrum resistance further comprises resistance to *Peronospora effusa* race Pe:19.

Embodiment 6 is the *Spinacia oleracea* plant of Embodiment 5, wherein the broad-spectrum resistance further comprises resistance to at least one *Peronospora effusa* race selected from the group consisting of Pe:6, Pe:8, and Pe:13.

Embodiment 7 is the *Spinacia oleracea* plant of Embodiment 6, wherein the broad-spectrum resistance further comprises resistance to at least one *Peronospora effusa* isolate selected from the group consisting of 254 and 381.

Embodiment 8 is the *Spinacia oleracea* plant of any one of the previous Embodiments, defined as an inbred plant or a hybrid plant.

Embodiment 9 is the *Spinacia oleracea* plant of any one of the previous Embodiments, defined as an agronomically elite plant.

Embodiment 10 is the agronomically elite *Spinacia oleracea* plant of any one of the previous Embodiments, wherein a representative sample of seed comprising said recombinant chromosomal segment has been deposited under NCMA Accession No. 202210003.

Embodiment 11 is a seed that produces the plant of any one of Embodiments 1 to 10, wherein the seed comprises the recombinant chromosomal segment.

Embodiment 12 is a plant part of the plant of any one of Embodiments 1 to 10, wherein said plant part comprises the recombinant chromosomal segment.

Embodiment 13 is the plant part of Embodiment 12, wherein the plant part is selected from the group consisting of: an embryo, a meristem, a cotyledon, pollen, a leaf, an anther, a root, a pistil, a flower, and a cell.

Embodiment 14 is a recombinant DNA segment comprising an allele from *Spinacia tetrandra* conferring broad-spectrum resistance to *Peronospora effusa* and lacking all or some genetic loci that are genetically linked thereto in *Spinacia tetrandra.*

Embodiment 15 is the recombinant DNA segment of Embodiment 14, wherein said recombinant DNA segment comprises the sequence of marker M1 (SEQ ID NO:1), marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), marker M8 (SEQ ID NO:8), or marker M9 (SEQ ID NO:9).

Embodiment 16 is the recombinant DNA segment of Embodiment 14 or 15, wherein said recombinant DNA segment comprises the sequence of marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), or marker M8 (SEQ ID NO:8).

Embodiment 17 is the recombinant DNA segment of any one of Embodiments 14 to 16, further defined as comprising at least a first genetic locus from *Spinacia oleracea.*

Embodiment 18 is the recombinant DNA segment of any one of Embodiments 14 to 17, wherein a representative sample of seed comprising said DNA segment from *Spinacia tetrandra* has been deposited under NCMA Accession No. 202210003.

Embodiment 19 is the recombinant DNA segment of any one of Embodiments 14 to 18, further defined as comprised within a plant, plant part, plant cell, or seed.

Embodiment 20 is a method for producing an elite *Spinacia oleracea* plant with broad-spectrum resistance to *Peronospora effusa* comprising introgressing into said plant a *Peronospora effusa* resistance allele within a recombinant chromosomal segment flanked in the genome of said plant by marker M1 (SEQ ID NO:1) and marker M9 (SEQ ID NO:9) on chromosome 3, wherein said resistance allele confers to said plant broad-spectrum resistance to *Peronospora effusa* relative to a plant lacking said allele, and wherein said introgressing comprises marker-assisted selection.

Embodiment 21 is a method for producing an elite *Spinacia oleracea* plant with broad-spectrum resistance to *Peronospora effusa* comprising introgressing into said plant a *Peronospora effusa* resistance allele within a recombinant chromosomal segment flanked in the genome of said plant by marker M2 (SEQ ID NO:2) and marker M8 (SEQ ID NO:8) on chromosome 3, wherein said resistance allele confers to said plant broad-spectrum resistance to *Peronospora effusa* relative to a plant lacking said allele, and wherein said introgressing comprises marker-assisted selection.

Embodiment 22 is the method of Embodiment 20 or 21, wherein said introgressing comprises: a) selfing a plant comprising said recombinant chromosomal segment or crossing a plant comprising said recombinant chromosomal segment with a second *Spinacia oleracea* plant of a different genotype to produce one or more progeny plants; and b) selecting a progeny plant comprising said recombinant chromosomal segment.

Embodiment 23 is the method of Embodiment 22, wherein selecting a progeny plant comprises detecting nucleic acids comprising marker M1 (SEQ ID NO:1), marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), marker M8 (SEQ ID NO:8), or marker M9 (SEQ ID NO:9).

Embodiment 24 is the method of Embodiment 22 or 23, wherein selecting a progeny plant comprises detecting nucleic acids comprising marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), or marker M8 (SEQ ID NO:8).

Embodiment 25 is the method of any one of Embodiments 22 to 24, wherein the progeny plant is an F₂-F₆ progeny plant.

Embodiment 26 is the method of any one of Embodiments 22 to 25, wherein said introgressing further comprises backcrossing or assaying for said broad-spectrum resistance to *Peronospora effusa.*

Embodiment 27 is a *Spinacia oleracea* plant obtainable by the method of any one of Embodiments 20 to 26.

Embodiment 28 is a method of selecting a *Spinacia oleracea* plant exhibiting broad-spectrum resistance to *Peronospora effusa,* comprising: a) selfing the *Spinacia oleracea* plant of claim 1 or crossing the *Spinacia oleracea* plant of claim 1 with a second *Spinacia oleracea* plant of a different genotype to produce one or more progeny plants; and b) selecting a progeny plant comprising said recombinant chromosomal segment.

Embodiment 29 is the method of Embodiment 28, wherein selecting said progeny plant comprises detecting a marker locus genetically linked to said recombinant chromosomal segment.

Embodiment 30 is the method of Embodiment 29, wherein selecting said progeny plant comprises detecting a marker locus within or genetically linked to a chromosomal segment flanked in the genome of said plant by marker M1 (SEQ ID NO:1) and marker M9 (SEQ ID NO:9) on chromosome 3.

Embodiment 31 is the method of any one of Embodiments 28 to 30, wherein selecting said progeny plant comprises detecting a marker locus within or genetically linked to a chromosomal segment flanked in the genome of said plant by marker M2 (SEQ ID NO:2) and marker M8 (SEQ ID NO:8) on chromosome 3.

Embodiment 32 is the method of any one of Embodiments 28 to 31, wherein selecting a progeny comprises detecting nucleic acids comprising marker M1 (SEQ ID NO:1), marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), marker M8 (SEQ ID NO:8), or marker M9 (SEQ ID NO:9).

Embodiment 33 is the method of any one of Embodiments 28 to 32, wherein selecting a progeny comprises detecting nucleic acids comprising marker marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), or marker M8 (SEQ ID NO:8).

Embodiment 34 is the method of any one of Embodiments 28 to 33, wherein said progeny plant is an F₂-F₆ progeny plant.

Embodiment 35 is the method of any one of Embodiments 28 to 34, wherein producing said progeny plant comprises backcrossing.

Embodiment 36 is a cell according to Embodiment 13.

Embodiment 37 is a tissue culture comprising the cell of Embodiment 13.

Embodiment 38 is a food product comprising the harvested leaves of the *Spinacia oleracea* plant of any one of Embodiments 1 to 10.

### EXAMPLES

The following disclosed embodiments are merely representative of the invention which may be embodied in various forms. Thus, specific structural, functional, and procedural details disclosed in the following examples are not to be interpreted as limiting.

### Example 1. Identification of Downy Mildew Resistance in Spinach

The test utilized for screening spinach accessions for resistance to downy mildew originated from the "Guidelines for the Conduct of Tests for Distinctness, Uniformity, and Stability of *Spinacia oleracea* L.," UPOV Code: SPINA_OLE, CPVO-TP/055/7, published by the International Union for the Protection of New Varieties of Plants (UPOV). The protocol is found at section Ad. 18 in the Guidelines and is as follows:

### Maintenance of Races:

Races of *Peronospora effusa* (ex *Peronospora farinosa* f. sp. *spinaciae*) are maintained on living host plants, obtainable from Naktuinbouw (P.O. Box 40, NL-2370 AA, Roelofarendsveen, Netherlands, or at naktuinbouw.com), or plant material with spores stored at -20° C for a maximum of one year.

### Execution of Test:

The growth stage of plants should be first cotyledons/leaf, eleven-day-old plants. The temperature condition should be maintained as 15°C during day/12°C during night. The light conditions should be 15 hours light per day, after emergence. Plants should be grown in soil in pots or trays in a glasshouse or growth chamber. To produce inoculum, sporulating leaves taken from host plants that were infected seven days before, are thoroughly rinsed with sterile tap water (maximum 150 ml water per 224 plants). To inoculate plants, the spore suspension is filtered through cheesecloth and sprayed on test plants until the inoculum covers the leaves but does not run off. 150 ml of suspension is enough for up to 3 x 224 plants. Spore density should be 20,000 to 100,000 conidia/ml water. The spore suspension should be used fresh. At least 20 plants should be tested.

As spinach downy mildew is wind-bome, sporulating plants should be kept in closed containers or isolated chambers to prevent any cross-contamination. Furthermore, resistant controls are needed in each multiplication and in each test to ensure the race identity. Light and humidity conditions during seedling development and incubation are critical. Optimal humidity of approximately 80-90% RH allows plant growth and fungal growth; strong light inhibits spore germination and infection. The test should be carried out in wintertime with protection against direct sunshine. After inoculation, the plants should remain under plastic for three days. After this time, the plastic should be slightly raised during the daytime.

### Duration of test:

Time period for multiplication of spores: spores should be harvested 7 days after inoculation; Time from sowing to inoculation: 11 days; Time from inoculation to reading: 10 days.

### Evaluation of infection:

Resistance is usually complete; sometimes necrotic spots are visible as a result of infection. Some varieties may have a slightly lower level of resistance, showing for example a slight tip sporulation however should be considered as the resistance is present. Susceptible plants show varying degrees of sporulation. Sporulation is visible as a grey covering on leaves, starting on the more humid abaxial side. A set of differential varieties from the International Working Group on *Peronospora* (IWGP) is used for the identification of the race (worldseed.org/document/differential-sets-peronospora-effusa-pe-spinach).

Plants are evaluated for resistance when the cotyledons are fully open (approximately 10-15 days after sowing). The plants are sprayed with inoculum (concentration = 10 spores/mL) with the relevant race. Plants are inoculated twice with the second spray 24-48 hours after the initial inoculation. Following inoculation, the trays are covered with domed lids to maintain a high humidity for the plants and the daylength is reduced to 8 hours of light. Disease symptoms are evaluated 9-12 days after inoculation when the susceptible controls are clearly sporulating.

### Example 2. Identification of a Novel Allele Associated with Resistance to Downy Mildew

Accessions of *Spinacia tetrandra* that represent seed collected in the wild are highly heterogeneous. Accordingly, the genetic composition of a seed bag containing a wild *Spinacia sp.* accession is also likely to be highly heterogeneous and it is therefore possible that each individual seed in the accession has a unique genetic composition.

Plants from the accession identified as GB1860 were crossed to a susceptible tester line and the F₁ progeny plants were evaluated using the methods set forth in Example 1 to identify those plants exhibiting resistance to certain races of *Peronospora effusa.* An F₁ plant was identified which exhibited resistance to at least *Peronospora effusa* races Pe: 14- Pe: 17. At the time of screening, race Pe:17 was the newest official race of *Peronospora effusa.* Genetic and sequence characterization of this F₁ progeny plant was performed to develop markers to select for the *S. tetrandra* haplotype across the downy mildew resistance locus on chromosome 3. This novel resistance allele was found to be comprised within a region located on chromosome 3 between 395,121 bp and 1,774,344 bp of the public *S. oleracea* reference genome v1. In addition, marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), and marker M8 (SEQ ID NO:8) were identified as interstitial markers (FIG. 1).

Using marker-assisted selection, material fixed for the *S. tetrandra* haplotype of this novel allele was developed and tested to confirm resistance to *Peronospora effusa* races Pe:14- Pe:17. This allele was also shown to confer resistance to a number of other *Peronospora effusa* races, including Pe:6, Pe:8, Pe:13, Pe:18, and Pe:19 and is strongly believed to also confer resistance to one or more of the novel isolates of *Peronospora effusa* that are currently under evaluation by the IWGP. This includes, but is not limited to, the isolates currently designated as 254 and 381. The novel allele can be combined with other resistance alleles to produce hybrid spinach varieties which have resistance to *Peronospora effusa* races Pe:1- Pe:19.

Markers that can be used to track the novel resistance allele on chromosome 3 are shown in Table 1 below. It should be noted that the claimed *S. oleracea* plants may contain the donor (*S. tetrandra*) allele at all indicated markers, however for marker M1 (SEQ ID NO:1) and marker M9 (SEQ ID NO:9), the markers flanking the interval on chromosome 3, the plant may contain either the donor or the recurrent (reference) parent allele. For interstitial marker M2 (SEQ ID NO:2), marker M3 (SEQ ID NO:3), marker M4 (SEQ ID NO:4), marker M5 (SEQ ID NO:5), marker M6 (SEQ ID NO:6), marker M7 (SEQ ID NO:7), and marker M8 (SEQ ID NO:8), the favorable allele is the allele from the donor parent.

**Table 1. Markers for tracking the novel Peronospora effusa resistance allele from S. tetrandra on chromosome 3.**

| **Marker Name** | **SNP Position in *S*. *oleracea* Reference Genome (bp)** | ***S*. *tetrandra* Allele** | **Reference Allele** | **Marker Size (bp)** | **Position of SNP in Marker (bp)** | **Marker Sequence (SEQ ID NO)** |
|---|---|---|---|---|---|---|
| **M1** | 395,121 | G | A | 301 | 151 | 1 |
| **M2** | 580,405 | G | T | 301 | 151 | 2 |
| **M3** | 586,509 | A | G | 301 | 151 | 3 |
| **M4** | 708,700 | G | T | 301 | 151 | 4 |
| **M5** | 850,553 | T | G | 301 | 151 | 5 |
| **M6** | 894,117 | T | C | 301 | 151 | 6 |
| **M7** | 1,401,465 | A | G | 301 | 151 | 7 |
| **M8** | 1,467,108 | T | C | 301 | 151 | 8 |
| **M9** | 1,774,344 | T | G | 301 | 151 | 9 |

### Example 3. Analyzing DNA Sequence Variation in Descendants of GB1860

The genetic variability within the *S. tetrandra* accessions GB1860 and GB1861 was validated using a haplotype-based analysis to analyze the genotypes of the different introgressions obtained from the S. *tetrandra* accessions. Individual plants (seven F₁ hybrids and two inbred) were selected from lines developed from different seed selections obtained from accessions GB1860 and GB1861 crossed to plants of the susceptible *S. oleracea* line Viroflay. Plant tissue was collected and DNA was extracted from these tissue samples for DNA analysis. The genomic region that was analyzed consisted of the first 2.2 Mb of chromosome 3, and the individual plant samples were compared to samples from plants comprising the region introgressed from *S*. *tetrandra* described in WO 2015054339 A1, which spans from 711,828 bp to 1,413,783 bp on chromosome 3. One of the plants comprised a chromosomal region that was introgressed from accession GB1860 and comprised the downy mildew resistance allele of the present disclosure. The number of pairwise differences between each of the haplotypes and the results indicated that accessions GB 1860 and GB1861 are indeed bulk populations of unique seed selections. Furthermore, the downy mildew resistance allele described herein has a haplotype that is distinct from known *Peronospora effusa* resistance alleles, including those previously described herein. In the ~700kb interval, the novel downy mildew resistance allele showed pairwise differences from the other haplotypes ranging from 3610 sites to 4508 sites, which corresponds to 0.51% to 0.64% of the sites in this interval.

## Claims

1. A method of selecting a *Spinacia oleracea* plant or *Spinacia oleracea* seed, said method comprising:
a. detecting in a population of *Spinacia oleracea* plants or *Spinacia oleracea* seeds a *Spinacia oleracea* plant or *Spinacia oleracea* seed comprising an allele on chromosome 3 that confers broad-spectrum resistance to *Peronospora effusa* relative to a *Spinacia oleracea* plant lacking said allele and
b. selecting said *Spinacia oleracea* plant or *Spinacia oleracea* seed comprising said allele on chromosome 3 that confers broad-spectrum resistance to *Peronospora effusa.*

2. The method according to claim 1, wherein said allele is associated with any marker selected from the group consisting of M1, M2, M3, M4, M5, M6, M7, M8 and M9.

3. The method according to claim 1, wherein said allele is associated with any marker selected from the group consisting of M2, M3, M4, M5, M6, M7 and M8.

4. The method according to any of the preceding claims, wherein the broad-spectrum resistance comprises resistance to *Peronospora effusa* races Pe:14, Pe:15, Pe:16, and Pe:17.

5. The method according to claim 4, wherein said broad-spectrum resistance further comprises resistance against race Pe:19.

6. The method according to claim 4 or 5, wherein the broad-spectrum resistance further comprises resistance to at least one *Peronospora effusa* race selected from the group consisting of: Pe:6 and Pe:13.

7. The method according to any one of claims 4 to 6, wherein the broad-spectrum resistance further comprises resistance to at least one *Peronospora effusa* isolate selected from the group consisting of: 254 and 381.

8. The method according to any of the preceding claims, where the marker
M1 comprises a G at position 151 in SEQ ID NO 1;
M2 comprises a G at position 151 in SEQ ID NO 2;
M3 comprises a A at position 151 in SEQ ID NO 3;
M4 comprises a G at position 151 in SEQ ID NO 4;
M5 comprises a T at position 151 in SEQ ID NO 5;
M6 comprises a T at position 151 in SEQ ID NO 6;
M7 comprises a A at position 151 in SEQ ID NO 7;
M8 comprises a T at position 151 in SEQ ID NO 8; and
M9 comprises a T at position 151 in SEQ ID NO 9.

9. The method according to any of the preceding claims, wherein the *Spinacia oleracea* plant or *Spinacia oleracea* seed is a cultivated plant or a seed of a cultivated plant.

10. The method according to any of the preceding claims, wherein the *Spinacia oleracea* plant or *Spinacia oleracea* seed is an elite plant or a seed of an elite plant.

11. A plant identifiable by the method according to any one of claims 1 to 10.

12. A nucleic acid molecule comprising an allele conferring resistance to *Peronospora effusa,* which nucleic acid molecule is obtainable or can be obtained from *S*. *oleracea seed* deposited under NCMA Accession No. 202210003 and which nucleic acid molecule comprises at least one marker selected from the group consisting of M1, M2, M3, M4; M5, M6, M7, M8, and M9.

13. The nucleic acid molecule of claim 12, comprising at least one marker selected from the group consisting of M2, M3, M4, M5, M6, M7 and M8.

14. The nucleic acid molecule according to claim 12 or 13, wherein the allele conferring resistance to *Peronospora effusa* is obtained from a *S. tetrandra* species.

15. The nucleic acid molecule according to any one of claims 12 to 15, which nucleic acid molecule at its 5' and/or 3' end further comprises nucleic acid sequences originating from *S. oleracea.*

16. A nucleic acid marker having a nucleic acid sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9.

17. The nucleic acid marker according to claim 16, wherein said nucleic acid sequence is
SEQ ID NO 1, comprising a G at position 151;
SEQ ID NO 2, comprising a G at position 151;
SEQ ID NO 3, comprising a A at position 151;
SEQ ID NO 4, comprising a G at position 151;
SEQ ID NO 5, comprising a T at position 151;
SEQ ID NO 6, comprising a T at position 151;
SEQ ID NO 7, comprising a A at position 151;
SEQ ID NO 8, comprising a T at position 151; or
SEQ ID NO 9, comprising a T at position 151.
